# EUROPEAN PATENT SPECIFICATION

(11) **EP 0 426 343 B1**
(45) Date of publication and mention of the grant of the patent: **23.02.1994**
(21) Application number: 90311508.7
(22) Date of filing: 19.10.1990
(51) Int. Cl.: C07C 19/08, C07C 17/20

(54) **Process for manufacture of 1,1-dichlorotetrafluoroethane**
Verfahren zur Herstellung von 1,1-Dichlorotetrafluorethan
Procédé pour la préparation de 1,1-dichlorotetrafluoroéthane

(30) Priority: 30.10.1989 US 429126
(43) Date of publication of application: 08.05.1991
(73) Proprietor: E.I. DU PONT DE NEMOURS AND COMPANY, Wilmington Delaware 19898 (US)
(72) Inventor: Gumprecht, William Henry, Wilmington, Delaware 19808 (US); Longoria, John Mark, Rochester, Michigan 48063 (US); Christoph, Frank J., Elkton, Maryland 21921 (US)
(74) Representative: Woodcraft, David Charles

(56) References cited:
- EP-A- 0 317 981
- US-A- 2 005 708
- US-A- 2 005 713
- US-A- 2 824 900

## Description

### 1. Field of the Invention:

This invention relates to a process for the manufacture of 1,1-dichlorotetrafluoroethane, CF₃CCl₂F, also known as chlorofluorocarbon (CFC)-114a, substantially free of its isomer, 1,2-dichlorotetrafluoroethane, CClF₂CClF₂ (CFC-114), and more particularly to a process involving liquid-phase hydrofluorination of 1,1,1-trichlorotrifluoroethane, CF₃CCl₃ (CFC-113a).

### 2. Description of the Related Art:

1,1-Dichlorotetrafluoroethane (CFC-114a) is of interest as an intermediate to 1,1,1,2-tetrafluoroethane, CF₃CH₂F (HFC-134a) via catalytic hydrogenolysis of its carbon-chlorine bonds: Brit. 1,578,933 (1980).

The tetrafluoroethane is an environmentally-acceptable potential replacement for currently-employed commercial refrigerants, blowing agents, aerosol propellants and sterilants that are believed implicated in the destruction of stratospheric ozone.

It is highly desired that the 1,1-dichlorotetrafluoroethane employed in the hydrogenolysis route to HFC-134a has as low a content of 1,2-dichlorotetrafluoroethane as practicable since any residual 1,2-dichloro compound present will lead to the isomer 1,1,2,2-tetrafluoroethane, CHF₂CHF₂ (HFC-134). This isomer is objectionable as it contaminates the HFC-134a product since the two isomers boil only 7°C apart. Unfortunately, CFC-114a, as currently manufactured, is obtained as a minor component of a mixture with its CFC-114 isomer. Also, its precursor, CFC-113a, is obtained as a minor component of a mixture with its isomer, 1,1,2-trichlorotrifluoroethane, CClF₂CCl₂F (CFC-113). Since the boiling points of the two trichlorotrifluoroethanes and of the two dichlorotetrafluoroethanes differ only slightly from one another, separation by distillation on a commercial scale is economically impractical. The lower-boiling dichlorotetrafluoroethanes (boiling range of about 3-4°C), however, are readily separable from the trichlorotrifluoroethanes (boiling range of about 46-48°C). One well-known and widely-used route to the trichlorotrifluoroethanes and dichlorotetrafluoroethanes involves reaction of hydrogen fluoride (HF) with tetrachloroethylene (C₂Cl₄) plus chlorine, or with its chlorine addition product, hexachloroethane (C₂Cl₆), in the liquid phase in the presence of an antimony pentahalide as catalyst: Henne, U.S. 1,978,840 (1934); Henne, U.S. 2,007,198 (1935); Daudt et al., U.S. 2,005,708 (1935); Daudt et al., U.S. 2,062,743 (1936); and, in particular, Benning, U.S. 2,478,362 (1949). Hamilton; in "Advances In Fluorine Chemistry", Volume 3, edited by Stacy et al., Washington, Buttersworth (1963), states, at pages 148 and 149, that the liquid-phase hydrofluorination route is well adapted to the production of the trichlorotrifluoroethanes (C₂Cl₃F₃) and the dichlorotetrafluoroethanes (C₂Cl₂F₄) among other major fluorocarbons. He also discloses, at page 122, Table 3, that the commercial C₂Cl₃F₃ and C₂Cl₂F₄ products of the organic chemicals industry consist predominantly of the more symmetrical isomers, that is, CClF₂CCl₂F (CFC-113) and CClF₂CClF₂ (CFC-114), respectively, the symmetrical term referring to the distribution of the fluorine radicals in the molecule.

The preparation of the trichlorotrifluoroethanes and the dichlorotetrafluoroethanes by vapor-phase reaction of HF with (A) C₂Cl₄+Cl₂ or (B) CClF₂CCl₂F over a suitable catalyst at elevated temperatures has also been well-documented in the art: Clark et al., U.S. 3,157,707 (1964) with (A) and a Cr₂O₃ catalyst; Swamer et al., U. S. 3,258,500 (1966) with (B) and a heat-activated Cr(III) oxide catalyst; Firth, U.S. 3,755,477 (1973) with (B) and a steam-treated Cr(III) oxide catalyst; Scherer et al., U.S. 3,752,850 (1973) with (A) over a Cr oxyfluoride catalyst; Christoph, U.S. 3,632,834 (1972) with (A), (B) and the like over a CrF₃ catalyst; Vecchio et al., U.S. 3,650,987 (1972) with (B) over an aluminum fluoride containing Fe, Cr and preferably also Ni halides or oxyhalides; Japanese Kokai No. SHO 51 (1976)-59804 from (B) using TiCl₃ on alumina and Foulletier, U.S. 4,439,534 (1984) and U.S. 4,474,895 (1984) from (B) using modified Cr(III) oxide catalysts. As specifically disclosed in the aforementioned Clark et al., Christoph, Vecchio et al., Foulletier and Japanese patents, the vapor-phase processes to the C₂Cl₃F₃ and C₂Cl₂F₄ compounds, whatever the catalyst employed, produce mixtures of the isomers, with the more symmetrical isomer predominating. Thus, it is clear that neither the liquid- nor the vapor-phase fluorination process is suitable for producing the desired asymmetric CFC-114a substantially free of its symmetric CFC-114 isomer from the normally-employed starting materials.

J. Calfee et al., U.S. 2,755,313 (1951), discloses HF fluorination of mixtures of C₂Cl₃F₃ isomers over an AlF₃ catalyst in the vapor phase to produce mixtures of C₂Cl₂F₄ isomers. It is also known that CFC-113 is isomerizable to CFC-113a [Miller, U.S. 2,598,411 (1952); Hauptschein et al., U.S. 3,087,974 (1963) and Hellberg et al., Ger. 1,668,346 (1971)], so that the isomerization of CFC-113 to CFC-113a represents a possibly suitable route to a raw material for the desired CFC-114a. On the other hand, no practical method for isomerizing, CFC-114 to CFC-114a exclusively appears available. It has been confirmed that attempts to produce CFC-114a free of CFC-114 by employing CFC-113a, in place of CFC-113, in a vapor-phase reaction with HF over a Cr(III) oxide catalyst, results in a CFC-114a product contaminated with undesirably high levels of CFC-114 and accompanied by the overfluorinated CF₃CF₂Cl (CFC-115), particularly at the elevated (300°-400°C) temperatures normally employed in vapor-phase fluorinations for the production of the tetrafluoro compounds. In addition, the unreacted CFC-113a component of the product stream also contained a substantial proportion of the isomeric CFC-113. The isomerized dichlorotetrafluoro- and the trichlorotrifluoro- contaminants are evidently the result of competing isomerization reactions under the stringent conditions employed.

EPA-0317981 describes a process for producing 1,1-dichloro-1,2,2,2-tetrafluoroethane, in which 1,1,2-trichloro-1,2,2-trifluoroethane is first isomerized to form 1,1,1-trichloro-2,2,2-trifluoroethane and the product fluorinated with hydrofluorine acid. When the process is carried out continuously, it is carried out in the gas phase.

### SUMMARY OF THE INVENTION

In view of the prior art, the present invention provides a catalytic, liquid-phase fluorination process for converting 1,1,1-trichlorotrifluoroethane to 1,1-dichlorotetrafluoroethane in the presence of 1,1,2-trichlorotrifluoroethane without the production of the undesirable isomer, 1,2-dichlorotetrafluoroethane or over-fluorinated products. According to the present invention the fluorination process can be conducted in a continuous manner where a feed mixture consisting essentially of the isomers CF₃CCl₃ and CClF₂CCl₂F, and optionally containing a minor proportion of CF₃CCl₂F, is continuously fed, along with a feed mixture consisting essentially of HF and a minor proportion of Cl₂ sufficient to maintain the antimony catalyst in the pentavalent (i.e., Sb⁺⁵) state, to a reactor containing the catalyst under controlled temperatures and pressures, and a product stream containing the desired CF₃CCl₂F substantially free of CClF₂CClF₂ is continuously removed from the reactor. The invention is based on the discovery that catalytic hydrofluorination of CF₃CCl₃ to CF₃CCl₂F can be effected to the substantial exclusion of reaction to its CClF₂CClF₂ isomer, even in the presence of substantial proportions of CClF₂CCl₂F, provided the reaction temperature is maintained at an effective CF₃CCl₃ fluorination temperature that is below the temperature at which CClF₂CCl₂F is fluorinated to CClF₂CClF₂, and preferably at a temperature at which CClF₂CCl₂F isomerizes to CF₃CCl₃. By such process, CF₃CCl₂F is readily produced and recovered containing less than 0.5 weight percent (5,000 ppm) of its isomer, even less than 0.1 weight percent (1,000 ppm), and under preferred conditions, essentially none of the isomer. Further, the process substantially completely avoids overfluorination to CFC-115 and CF₃CF₃ (FC-116).

Thus, the present invention provides a process for producing 1,1-dichlorotetrafluoroethane substantially free of 1,2-dichlorotetrafluoroethane comprising the steps of:
(a) contacting
   1,1,1-trichlorotrifluoroethane with at least a molar proportion of hydrogen fluoride in the presence of a catalytically effective amount of an antimony pentahalide characterized by the formula SbCl₅₋ₓFₓ, where x is 0 to 3, at a temperature that is sufficiently high to convert 1,1,1-trichlorotrifluoroethane to 1,1-dichlorotetrafluoroethane but is below the temperature at which 1,1,2-trichlorotrifluoroethane is substantially converted to 1,2-dichlorotetrafluorethane, and at a pressure sufficient to maintain at least a portion of the 1,1,1-trichlorotrifluoroethane in the liquid state, thereby forming a liquid reaction medium; and
(b) recovering from the reaction medium 1,1-dichlorotetrafluoroethane substantially free of 1,2-dichlorotetrafluoroethane.

It is an object of this invention to provide a process for hydrofluorinating CF₃CCl₃ to CF₃CCl₂F substantially free of CClF₂CClF₂. It is another object to provide such a process that avoids the production of over-fluorinated products. It is a further object to provide a process as above that selectively fluorinates CF₃CCl₃, in the presence of CClF₂CCl₂F, to CF₃CCl₂F substantially without fluorinating the CClF₂CCl₂F to CClF₂CClF₂. It is yet a further object to provide such a fluorination process that not only converts CF₃CCl₃ to CF₃CCl₂F in the presence of CClF₂CCl₂F, but which results in some isomerization of CClF₂CCl₂F to CF₃CCl₃.

Fulfillment of these objects and the presence and fulfillment of additional objects will be apparent upon complete reading of the specification and attached claims.

### DESCRIPTION OF THE PREFERRED EMBODIMENTS

The overall process according to the present invention can be conducted either batchwise or preferably continuously, in a manner generally known to the art for conducting catalyzed liquid-phase hydrofluorination reactions. The reactants are to be maintained at temperatures and pressures as defined. More specifically, temperature and pressure will be controlled such that at least a portion of the 1,1,1-trichlorotrifluoroethane feed stock is in the liquid state in the reaction zone, and a substantial proportion of the 1,1-dichlorotetrafluoroethane reaction product is in the gaseous state, so that the dichlorotetrafluoroethane product and the HCl by-product of the fluorination reaction can be conveniently removed from the reaction zone. The CF₃CCl₃ reactant can be employed in the essentially pure state, i.e., containing substantially no CClF₂CCl₂F, or as a mixture with its isomer. Although the CClF₂CCl₂F content of the mixture can vary widely, it will normally correspond to a minor amount, preferably less than about 10%, more preferably less than about 2%, still more preferably less than about 1% by weight of the CF₃CCl₃ component, and normally about 0.1% by weight. Included are CF₃CCl₃-CClF₂CCl₂F mixtures produced by isomerizing CClF₂CCl₂F to CF₃CCl₃ as described in the art.

The CF₃CCl₃ reactant, alone or together with its isomer, may also contain CF₃CCl₂F as a by-product of the aforementioned isomerization process, generally in minor proportions.

The antimony pentahalide catalyst, SbCl₅₋ₓFₓ where x = 0 to 3, is conveniently SbCl₅ (x = O) but may be an antimony pentachlorofluoride, where x = 1 to 3, more usually 1 to 2 including mixtures of the pentafluoride and pentachloride, which may be preformed or formed in situ during the course of the reaction, as is well-known in the art. The quantity of catalyst can vary widely provided it is a catalytically-effective amount which is readily determined by trial.

The HF/CF₃CCl₃ feed ratio can also vary widely, but will normally be at least equimolar with respect to the CF₃CCl₃ reactant and usually not more than 10/1 for economic reasons. Preferably, the HF/CF₃CCl₃ ratio is in the range 1.5/1 to 3/1.

The reaction temperature, which will be below the temperature at which CClF₂CCl₂F is fluorinated to CClF₂CClF₂ in the presence of hydrogen fluoride and the antimony pentahalide catalyst, will normally be below about 130°C but at least about 80°C. Preferably, the reaction temperature is in the range of from about 90°C to about 125°C, more preferably from about 100° to about 120°C. At temperatures lower than 80°C, the CFC-114a production rate is lower than desired, at temperatures higher than 130°C, greater than desired amounts of CFC-114 begin to be observed.

The process can be conducted at atmospheric, subatmospheric or superatmospheric pressure, with at least atmospheric pressure preferred. Pressures above atmospheric are particularly preferred and are coordinated with the reaction temperatures such that at least a portion of the trichlorotrifluoroethane feed is maintained in the liquid state, and the dichlorotetrafluoroethane reaction product is in the vapor state. For example, at temperatures in the 80° to 130°C range, the pressure will be in the 1035 KPa (150 psig) to 1725 KPa (250 psig) range. Pressures are conveniently controlled with pressure relief valves as is conventional in the chemical process art.

The pentavalent antimony halide catalyst tends to dissociate into trivalent antimony halide and chlorine, and this increases with increasing temperature. Thus, Cl₂ gas tends to leave the reaction mixture and be swept out of the reactor in the gaseous effluent, particularly under continuous process conditions. For this reason, it is generally advisable to incorporate Cl₂ gas into the HF feed stream in amounts sufficient to maintain the antimony catalyst substantially in the pentavalent state. The amount of Cl₂ added can be controlled to match the amount that appears in the product stream issuing from the reactor.

As the reaction proceeds, under the mixed trichlorotrifluoroethane embodiment of the invention, 1,1,2-trichlorotrifluoroethane tends to build up in the liquid reaction mixture phase as the 1,1,1-trichlorotrifluoroethane is converted to 1,1-dichlorotetrafluoroethane. Surprisingly, under the defined conditions, the 1,1,2-trichloro isomer remains unreacted as little or no 1,2-dichlorotetrafluoroethane appears in the reaction product stream. Surprisingly, too, some of the 1,1,2-trichloro compound is isomerized to the 1,1,1-trichloro compound which, in turn, is converted to the desired 1,2-dichlorotetrafluoroethane.

The reaction product stream can be treated in accordance with any of the techniques known to the art for separating the desired fluorination product from by-product hydrogen chloride, unreacted hydrogen fluoride, if any, chlorine gas, if any, and any trichlorotrifluoroethane that may have been volatilized from the reaction mixture. The product stream, for example, may be scrubbed with water or aqueous alkali to remove hydrogen halides and chlorine, dried with a drying agent, such as silica gel or a molecular sieve adapted to such purpose, then condensed and recovered. The dichlorotetrafluoroethane product, consisting essentially of the 1,1-dichloro isomer substantially free of the 1,2-dichloro isomer, is readily separated from any trichlorotrifluoroethanes by distillation in view of the substantial difference in their boiling points. The trichlorotrifluoroethanes can be recycled to the reactor, if desired.

The reactor and its associated feed lines, effluent line and associated units should be constructed of materials resistant to hydrogen fluoride, hydrogen chloride, chlorine and the antimony halide catalyst material. Typical materials of construction, well-known to the fluorination art, include stainless steels, in particular of the austentitic type, and the well-known high nickel alloys, such as "MONEL" nickel-copper alloys, "HASTELLOY" nickel-based alloys and, "INCONEL" nickel-chromium alloys. Also suitable for reactor fabrication are such polymeric plastics as polytrifluorochloroethylene and polytetrafluoroethylene, generally used as linings.

The following examples are presented to further illustrate specific embodiments of the present invention. Each example utilized a stainless-steel reactor consisting essentially of a 57 litres (15-gallon) reaction vessel equipped with a heating means, trichlorotrifluoroethane, HF and Cl₂ feed lines, a condenser/dephlegmator to control effluent temperature and return unreacted HF, CF₂ClCFCl₂ and CF₃CCl₃ to the reactor, and a pressure relief valve to control reaction pressure. The reaction product stream was analyzed by means of gas chromatography. The gas chromatograph consisted of a 6.1 metres (20-foot) column containing "KRYTOX" perfluorinated ether on an inert support ("CARBOPAK" B) and packed in 3.18 mm (1/8 inch) stainless steel tubing. The carrier gas was N₂ at a flow rate of 50 cc/minute. A flame ionization detector was used. The effluent gas and reaction mass contents, where given, are in weight percents as determined gas-chromatographically.

### Example I

A mixture of 1,1,1-trichlorotrifluoroethane (CFC-113a) containing 0.71% 1,1,2-trichlorotrifluoroethane (CFC-113) and 1.75% 1,1,-dichlorotetrafluoroethane (CFC-114a) was fed, along with a separate feed of 1.8 moles of HF and 0.036 mole of Cl₂ per mole of CFC-113a, to the pressure reactor (described above) containing 66.7 kilos (147 lbs); (222 grams-mole) (0.49 lb-mole) of SbCl₅. The CFC-113a and HF/Cl₂ feeds were continuously passed through dip legs into the SbCl charge at feed rates corresponding to 8.16 kilograms (18 lbs); 43.5 gram-mole/hour) (0.096 lb-mole)/hour of the CFC-113a feed, 1.59 kilograms (3.5 lbs), 79.4 gram/mole/hour) (0.175-lb-mole/hour) of HF and 113.4 grams (0.25 lb), 1.6 gram-mole/hr (0.0035 lb-mole/ hour of Cl₂.

The temperature of the reaction mixture was maintained at 110°C, and the reactor pressure at 1725 KPa (250 psig). The effluent gas exiting the reaction zone was maintained at 73°C by means of the dephlegmator, and was removed continuously. Steady state, achieved after 18 hours of operation, was determined by analyses of the effluent gas and samples of reaction mass taken periodically. The organic component of the effluent gas was found to consist, in area %, of:
1,1-dichlorotetrafluoroethane (99.5%)
1,2-dichlarotetrafluoroetgane (0.10%)
1,1,1-trichlorotrifluoroethane (0.30%)
1,1,2-trichlorotrifluoroethane (0.10%)

The volatile inorganics, consisting of by-product HCl, some unreacted HF and small amounts of Cl₂. were not quantified. Conversion of the CFC-113a/CFC-113 feed was 99.6%, and the yield of CFC-114a was 98.2% when that in the feed is accounted for.

The samples of reaction mixture taken from the reactor showed that, at steady state, the organic component consisted essentially of CFC-113a containing 27% CFC-113, which concentration remained constant throughout the run which was terminated after 9.5 hours of steady-state operation.

### Example II

The procedure of Example I was repeated except that 145 gram-mole (0.32 lb-mole) of SbCl₅ was used, the CFC-113a feed rate was 41.3 gram-mole/hr (0.091 lb-mole/hr), the reaction temperature was 130°C and the pressure was 1517 KPa (220 psig). The duration of the run was 18 hours. The off-gas. contained CFC-114a in which no CFC-114 was detected. The conversion of the CFC-113a/CFC-113 feed was 99.5%, and the yield of CFC-114a was 98.2% when that in the feed is accounted for.

The higher operating temperature in Example II increased the rates of all reactions to varying degrees. Evidently, CFC-113 isomerizes to CFC-113a faster than it fluorinates to CFC-114 under the Example II conditions since no detectable amount of CFC-114 was generated and, in contrast to Example I, CFC-113 did not accumulate in the system.

## Claims

1. A process for producing 1,1-dichlorotetrafluoroethane substantially free of 1,2-dichlorotetrafluoroethane comprising the steps of:
(a) contacting 1,1,1-trichlorotrifluoroethane with at least a molar proportion of hydrogen fluoride in the presence of a catalytically effective amount of an antimony pentahalide characterized by the formula SbCl₅₋ₓFₓ, where x is 0 to 3, at a temperature that is sufficiently high to convert 1,1,1-trichlorotrifluoroethane to 1,1-dichlorotetrafluoroethane but is below the temperature at which 1,1,2-trichlorotrifluoroethane is substantially converted to 1,2-dichlorotetrafluoroethane, and at a pressure sufficient to maintain at least a portion of the 1,1,1-trichlorotrifluoroethane in the liquid state, thereby forming a liquid reaction medium; and
(b) recovering from said reaction medium 1,1-dichlorotetrafluoroethane containing less than 0.5 weight percent of 1,2-dichlorotetrafluoroethane.

2. A process of Claim 1 wherein the 1,1-dichlorotetrafluoroethane being recovered is substantially free of over-fluorinated reaction products.

3. A process of Claim 2 wherein the pressure is insufficient to condense 1,1-dichlorotetrafluoroethane but sufficient to condense at least a portion of the 1,1,1-trichlorotrifluoroethane.

4. A process of Claim 1, 2 or 3 wherein said contacting of 1,1,1-trichlorotrifluoroethane with hydrogen fluoride is in the presence of 1,1,2-trichlorotrifluoro-ethane.

5. A process of Claim 4 wherein the temperature is from 80°C to 130°C.

6. A process of Claim 5 wherein the temperature is from 100°C to 120°C.

7. A process of Claim 5 wherein the pressure is from 1035 KPa (150 psig) to 1725 KPa (250 psig).

8. A process of any one of the preceding claims further comprising the step of adding to the reaction medium an effective amount of chlorine such as to maintain the antimony in the pentavalent state.

9. A process of Claim 1 for producing 1,1-dichlorotetrafluoroethane continuously substantially free of 1,2-dichlorotetrafluoroethane and over-fluorinated products comprising the steps of:
(a) providing a temperature- and pressure- controlled reaction vessel charged with an effective amount of said antimony pentahalide catalyst;
(b) establishing within said reaction vessel and in contact with said catalyst a liquid phase trichlorotrifluoroethane-containing reaction medium;
(c) continuously adding to said reaction medium a feed stream of trichlorotrifluoroethane reactant and a molar excess of hydrogen fluoride; and
(d) continuously removing from said reaction vessel a gaseous product stream containing 1,1-dichlorotetrafluoroethane containing less than 0.5 weight percent of 1,2-dichlorotetrafluoroethane and substantially free of over-fluorinated products.

10. A process of Claim 9 further comprising the step of continuously adding an effective amount of chlorine to said reaction medium such as to maintain the antimony in the pentavalent state.

11. A process of Claim 10 further comprising the steps of:
(a) separating and recovering said 1,1-dichlorotetrafluoroethane from said gaseous products; and
(b) separating and recycling to said reaction vessel any unreacted trichlorotrifluoroethane.

12. A process of any one of Claims 9 to 11 which is conducted at a temperature of from 80°C to 130°C and at a pressure of from 1035 KPa (150 psig) to 1725 KPa (250 psig).

13. A process of any one of Claims 9 to 12 wherein the ratio of hydrogen fluoride to 1,1,1-trichlorotrifluoroethane is from 1.5:1 to 3:1.

14. A process of any one of Claims 9 to 13 wherein the catalyst is formed in situ in the reaction vessel.

15. A process of any one of Claims 9 to 14 wherein the 1,1,1-trichlorotrifluoroethane contains less than 10% 1,1,2-trichlorotrifluoroethane.

## Patentansprüche

1. Verfahren zur Herstellung von 1,1-Dichlortetrafluorethan, im wesentlichen frei von 1,2-Dichlortetrafluorethan, umfassend die Stufen:
(a) Kontaktieren von 1,1,1-Trichlortrifluorethan mit mindestens einem molaren Anteil von Fluorwasserstoff in Gegenwart einer katalytisch wirksamen Menge eines Antimonpentahalogenids, gekennzeichnet durch die Formel SbCl₅₋ₓFₓ, worin x eine Zahl von 0 bis 3 bedeutet, bei einer Temperatur, die hoch genug ist, um 1,1,1-Trichlortrifluorethan in 1,1-Dichlortetrafluorethan umzuwandeln, jedoch unter der Temperatur liegt, bei der 1,1,2-Trichlortrifluorethan im wesentlichen in 1,2-Dichlortetrafluorethan umgewandelt wird, und bei einem Druck, der ausreicht, um mindestens einen Teil von 1,1,1-Tri-chlortrifluorethan in flüssigem Zustand zu halten, wodurch ein flüssiges Reaktionsmedium gebildet wird; und
(b) Gewinnen von 1,1-Dichlortetrafluorethan, das weniger als 0,5 Gew.-% 1,2-Dichlortetrafluorethan enthält, aus dem genannten Reaktionsmedium.

2. Verfahren nach Anspruch 1, bei dem das 1,1-Dichlortetrafluorethan im wesentlichen frei von überfluorierten Reaktionsprodukten gewonnen wird.

3. Verfahren nach Anspruch 2, bei dem der Druck nicht ausreicht, um 1,1-Dichlortetrafluorethan zu kondensieren, jedoch ausreicht, um mindestens einen Teil des 1,1,1-Trichlortrifluorethans zu kondensieren.

4. Verfahren nach Anspruch 1, 2 oder 3, bei dem das genannte Kontaktieren von 1,1,1-Trichlortrifluorethan mit Fluorwasserstoff in Gegenwart von 1,1,2-Trichlortrifluorethan stattfindet.

5. Verfahren nach Anspruch 4, bei dem die Temperatur 80 °C bis 130 °C beträgt.

6. Verfahren nach Anspruch 5, bei dem die Temperatur 100 °C bis 120 °C beträgt.

7. Verfahren nach Anspruch 5, bei dem der Druck 1035 KPa (150 psig) bis 1725 KPa (250 psig) beträgt.

8. Verfahren nach einem der vorgenannten Ansprüche, das weiterhin umfaßt die Stufen Zugeben zu dem Reaktionsmedium einer wirksamen Menge Chlor, so daß das Antimon im fünfwertigen Zustand verbleibt.

9. Verfahren nach Anspruch 1 zur Herstellung von 1,1-Dichlortetrafluorethan kontinuierlich im wesentlichen frei von 1,2-Dichlortetrafluorethan und überfluorierten Produkten, umfassend die Stufen:
(a) Bereitstellen eines temperatur- und druckkontrollierten Reaktionsgefäßes, gefüllt mit einer wirksamen Menge des genannten Antimonpentahalogenid-Katalysators;
(b) Herstellen in dem genannten Reaktionsgefäß und in Kontakt mit dem genannten Katalysator eines Trichlortrifluorethan-haltigen Flüssigphasen-Reaktionsmediums;
(c) kontinuierlich Zuführen eines Einspeisestroms aus Trichlortrifluorethan-Reagens und einem molaren Überschuß Fluorwasserstoff zu dem genannten Reaktionsmedium; und
(d) kontinuierlich Entfernen aus dem genannten Reaktionsgefäß eines gasförmigen Produktstroms, enthalten 1,1-Dichlortetrafluorethan, enthaltend weniger als 0,5 Gew.-% 1,2-Dichlortetrafluorethan, im wesentlichen frei von überfluorierten Produkten.

10. Verfahren nach Anspruch 9, das weiterhin umfaßt die Stufe kontinuierlich Zugeben einer wirksamen Menge von Chlor zu dem genannten Reaktionsmedium, so daß das Antimon im fünfwertigen Zustand verbleibt.

11. Verfahren nach Anspruch 10, das ferner umfaßt die Stufen:
(a) Trennen und Gewinnen des genannten 1,1-Dichlortetrafluorethans aus den genannten gasförmigen Produkten; und
(b) Trennen und Rückführen in das genannte Reaktionsgefäß von jeglichem nicht umgesetzten Trichlortrifluorethan.

12. Verfahren nach einem der Ansprüche 9 bis 11, das bei einer Temperatur von 80 °C bis 130 °C und einem Druck von 1035 KPa (150 psig) bis 1725 KPa (250 psig) durchgeführt wird.

13. Verfahren nach einem der Ansprüche 9 bis 12, bei dem das Verhältnis von Fluorwasserstoff zu 1,1,1-Trichlortrifluorethan 1,5:1 bis 3:1 beträgt.

14. Verfahren nach einem der Ansprüche 9 bis 13, bei dem der Katalysator in situ in dem Reaktionsgefäß gebildet wird.

15. Verfahren nach einem der Ansprüche 9 bis 14, bei dem das 1,1,1-Trichlortrifluorethan weniger als 10 % 1,1,2-Trichlortrifluorethan enthält.

## Revendications

1. Procédé de préparation de 1,1-dichlorotétrafluoroéthane pratiquement exempt de 1,2-dichlorotétrafluoroéthane, comprenant les étapes de:
(a) mise en contact de 1,1,1-trichlorotrifluoroéthane avec au moins une proportion molaire de fluorure d'hydrogène, en présence d'une quantité catalytiquement efficace d'un pentahalogénure d'antimoine caractérisé par la formule SbCl₅₋ₓFₓ, où X est 0 à 3, à une température qui est suffisamment élevée pour convertir le 1,1,1-trichlorotrifluoroéthane en 1,1-dichlorotétrafluoro-éthane mais qui est en dessous de la température à laquelle le 1,1,2-trichlorotrifluoroéthane est pratiquement converti en 1,2-dichlorotétrafluoro-éthane, et à une pression suffisante pour maintenir au moins une partie du 1,1,1-trichlorotrifluoroéthane à l'état liquide, formant ainsi un milieu réactionnel liquide; et
(b) récupération à partir dudit milieu réactionnel du 1,1-dichlorotétrafluoroéthane contenant moins de 0,5 pour cent en poids de 1,2-dichlorotétrafluoroéthane.

2. Procédé selon la revendication 1, dans lequel le 1,1-dichlorotétrafluoroéthane récupéré est pratiquement exempt de produits réactionnels surfluorés.

3. Procédé selon la revendication 2, dans lequel la pression est insuffisante pour condenser le 1,1-dichlorotétrafluoroéthane mais suffisante pour condenser au moins une partie du 1,1,1-trichlorotrifluoroéthane.

4. Procédé selon la revendication 1, 2 ou 3, dans lequel ladite mise en contact du 1,1,1-trichlorotrifluoroéthane avec le fluorure d'hydrogène est effectuée en présence de 1,1,2-trichlorotrifluoroéthane.

5. Procédé selon la revendication 4, dans lequel la température est de 80°C à 130°C.

6. Procédé selon la revendication 5, dans lequel la température est de 100°C à 120°C.

7. Procédé selon la revendication 5, dans lequel la pression relative est de 1035kPa à 1725kPa.

8. Procédé selon l'une quelconque des revendications précédentes, comprenant en outre l'étape d'addition au milieu réactionnel d'une quantité efficace de chlore de façon à maintenir l'antimoine à l'état pentavalent.

9. Procédé selon la revendication 1 pour la préparation de 1,1-dichlorotétrafluoroéthane, en continu, pratiquement exempt de 1,2-dichlorotétrafluoroéthane et de produits surfluorés, comprenant les étapes de:
(a) fourniture d'un récipient de réaction à température et pression régulées, chargé avec une quantité efficace dudit catalyseur pentahalogénure d'antimoine;
(b) établissement à l'intérieur dudit récipient de réaction, et en contact avec ledit catalyseur, d'un milieu réactionnel en phase liquide contenant du trichlorotrifluoroéthane;
(c) addition en continu audit milieu réactionnel d'un flux d'alimentation de réactif trichlorotrifluoroéthane, et d'un excès molaire de fluorure d'hydrogène; et
(d) élimination en continu dudit récipient de réaction d'un courant de produits gazeux contenant du 1,1-dichlorotétrafluoroéthane contenant moins de 0,5 pour cent en poids de 1,2-dichlorotétrafluoroéthane et pratiquement exempt de produits surfluorés.

10. Procédé selon la revendication 9, comprenant en outre l'étape d'addition en continu d'une quantité efficace de chlore audit milieu réactionnel de façon à maintenir l'antimoine à l'état pentavalent.

11. Procédé selon la revendication 10, comprenant en outre les étapes de:
(a) séparation et récupération dudit 1,1-dichlorotétrafluoroéthane à partir desdits produits gazeux; et
(b) séparation et recyclage vers ledit récipient de réaction de tout trichlorotrifluoroéthane n'ayant pas réagi.

12. Procédé selon l'une quelconque des revendications 9 à 11, qui se déroule à une température de 80°C à 130°C et à une pression relative de 1035kPa à 1725kPa.

13. Procédé selon l'une quelconque des revendications 9 à 12, dans lequel le rapport du fluorure d'hydrogène au 1,1,1-trichlorotrifluoroéthane est de 1,5:1 à 3:1.

14. Procédé selon l'une quelconque des revendications 9 à 13, dans lequel le catalyseur est formé in situ dans le récipient de réaction.

15. Procédé selon l'une quelconque des revendications 9 à 14, dans lequel le 1,1,1-trichlorotrifluoroéthane contient moins de 10% de 1,1,2-trichlorotrifluoroéthane.
